(19) Europäisches Patentamt / European Patent Office / Office européen des brevets

(11) EP 3 539 561 A1

# (12) EUROPEAN PATENT APPLICATION
published in accordance with Art. 153(4) EPC

(43) Date of publication:
**18.09.2019 Bulletin 2019/38**

(21) Application number: **17869580.5**

(22) Date of filing: **09.11.2017**

(51) Int Cl.:
***A61K 38/52** (2006.01)* ***A61K 35/12** (2015.01)*
***A61K 35/76** (2015.01)* ***A61K 35/761** (2015.01)*
***A61K 48/00** (2006.01)* ***A61P 9/10** (2006.01)*

(86) International application number:
**PCT/JP2017/040395**

(87) International publication number:
**WO 2018/088464 (17.05.2018 Gazette 2018/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **11.11.2016 JP 2016220805**

(71) Applicants:
- **Stemrim Inc.**
  **Ibaraki-shi, Osaka 567-0085 (JP)**
- **Osaka University**
  **Suita-shi, Osaka 565-0871 (JP)**

(72) Inventors:
- **TAMAI, Katsuto**
  **Suita-shi**
  **Osaka 565-0871 (JP)**
- **YAMAZAKI, Takehiko**
  **Ibaraki-shi**
  **Osaka 567-0085 (JP)**
- **YOKOTA, Koichi**
  **Ibaraki-shi**
  **Osaka 567-0085 (JP)**
- **AOTO, Takahiro**
  **Ibaraki-shi**
  **Osaka 567-0085 (JP)**

(74) Representative: **Moré, Solveig Helga et al**
**Kroher Strobel**
**Rechts- und Patentanwälte PartmbB**
**Bavariaring 20**
**80336 München (DE)**

## (54) THERAPEUTIC AGENT FOR CEREBRAL INFARCTION

(57) The purpose of the present invention is to provide a novel medicine that is effective in treating cerebral infarction. The present invention provides a pharmaceutical composition that contains a peptidyl-prolyl cis-trans isomerase B (PPIB) protein, a nucleic acid encoding the PPIB protein, or a cell that secretes the PPIB protein.

[Fig. 2]

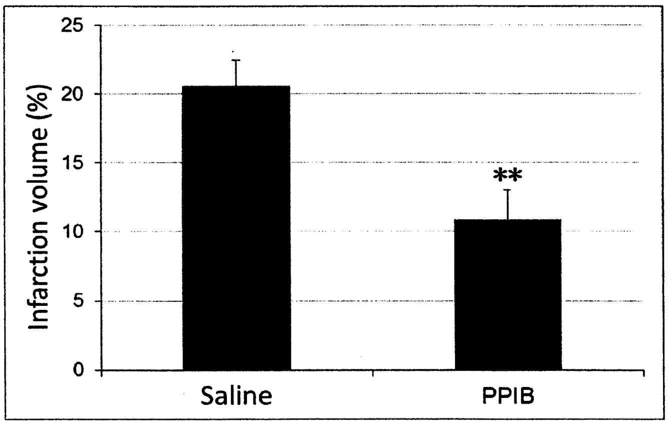




Printed by Jouve, 75001 PARIS (FR)

**Description**

Technical Field

The present invention relates to a pharmaceutical composition for treating cerebral infarction containing in vivo protein.

Background Art

Cerebral infarction is classified into cerebrovascular disease, so-called stroke, and 60% of the deaths of cerebrovascular disease (about 70,000 a year) have died as a cause of cerebral infarction (Non-Patent Document 1). Cerebral infarction often causes serious sequelae, such as paralysis of the limbs, speech problems, and memory problems, to return to society even if it does not lead to death. Thrombolysis is known as the best treatment because cerebral infarction is caused by a blood clot in a blood vessel in the brain. However, the drug t-PA (alteplase), which is used for thrombolytic therapy, can be used only for a patient within 4.5 hours after onset due to side effects and difficulties in regenerating nerve cells that have been ischemic for a long time. Thus, it has been estimated that 5 to 6% of all patients with cerebral infarction are actually applied t-PA in Japanese medical practice (Non-Patent Document 2). Thus, there is no effective treatment available for most patients. The therapeutic methods other than thrombolytic therapy include anticoagulation therapy (Non-Vitamin K Antagonist Oral Anticoagulant: dabigatran, etc.), and cerebroprotective therapy (antioxidants: edaravone). However, these have not been as effective as thrombolytic therapy. Therefore, the novel medicine effective for treatment of cerebral infarction has been demanded.

Human peptidyl-prolyl cis-trans isomerase B (hereinafter also referred to as PPIB, PPIase B, peptidylprolyl trans isomerase B), also called as cyclophilin B, has been cloned as a cyclosporin A-binding protein following peptidyl-prolyl cis-trans isomerase A (hereinafter referred to as PPIA) (Non-Patent Documents 3 and 4). It is known that PPIB has peptidyl-prolyl cis-trans isomerase activity, and that the activity is inhibited by cyclosporin A (CsA). In addition, PPIB is a protein consisting of 216 amino acids, and shows homology with PPIA in some domains. Moreover, unlike PPIA, there is a hydrophobic residue-rich region on the N-terminal of PPIB, and it is an ER-directed signal sequence, and thus, it is known as a feature of PPIB that PPIB is secreted extracellularly (Non-Patent Documents 3 and 5).

PPIA and PPIB have been reported to be important proteins for HIV-1 particle formation. PPIA and PPIB bind to capsid proteins during HIV-1 assembly and are incorporated into the virus. In the presence of PPIA and PPIB inhibitors such as CsA, it has been found that binding of PPIA and PPIB to capsid proteins is inhibited, and viral replication in host cell is also inhibited (Non-Patent Document 6).

PPIB has been found in the test that searches for a substance having migration activity for bone marrow mesenchymal stem cells contained in the supernatant of cultured breast cancer cells, and the migration activity is suppressed by an antibody against CD147, and thus it has been revealed that CD147 on the surface of bone marrow mesenchymal stem cell is the target receptor (Non-Patent Document 7).

As a role of PPIB to an inflammation, it has been reported that there is human neutrophil migration activity via CD147 (Non-Patent Document 8). The ligand binding site of PPIB has a central core region similar to that of PPIA. However, PPIB has an N-terminal binding site to glycosaminoglycans (GAGs) not present in PPIA. PPIB promotes adhesion of CD4+ CD45RO+ T cells to extracellular matrix via CD147, whereas PPIA lacks this activity, rather competes and suppresses it. It has been revealed that PPIB has, as an inflammatory factor, a function different from PPIA having chemokine-like activity (Non-Patent Documents 9 and 10).

While PPIA is known to promote a production of proinflammatory cytokine TNF-$\alpha$ in macrophage, PPIB has no such activity, rather it has been revealed that an increase in the production of TNF-$\alpha$ by LPS is suppressed by pretreating macrophages with PPIB (Non-Patent Document 11).

Thus, while PPIB has properties similar to those of PPIA in the structure, enzyme activity, target receptor, etc., it is also known that PPIB has unique properties. In particular, it is considered that various effects unique to PPIB are given on the inflammation.

In addition, it has been revealed that PPIB is involved in the folding of collagen I as a chaperone by the above-mentioned peptidyl-prolyl cis-trans isomerase activity, and it is considered that the lack of PPIB is one of the causes of bone formation failure (Non-Patent Documents 12 and 13).

As described above, PPIB is known to have multiple functions, but in a subject with specific disease, the effect of administration of PPIB on the body is unclear, and it is unknown whether PPIB may be used as a therapeutic agent for cerebral infarction.

Prior Art Documents

Non-Patent Documents

Non-Patent Document 1: Ministry of Health, Labour and Welfare, Summary of Population Survey Report (Definite) of 2015, Table 6.
Non-Patent Document 2: Neurological Surgery (December, 2015), Vol. 43, No. 12, pp. 1055-1070.
Non-Patent Document 3: Price ER et al., Proc Natl Acad Sci U S A. 1991 Mar 1;88(5):1903-7.
Non-Patent Document 4: Spik G et al., J Biol Chem. 1991 Jun 15;266(17):10735-8.
Non-Patent Document 5: Hasel KW et al., Mol Cell Biol. 1991 Jul;11(7):3484-91.
Non-Patent Document 6: Luban J et al., Cell. 1993 Jun 18;73(6) :1067-78.
Non-Patent Document 7: Lin SY et al., Exp Cell Res. 2008 Oct 15;314(17):3107-17.
Non-Patent Document 8: Yurchenko V et al., Biochem Biophys Res Commun. 2001 Nov 9;288(4):786-8.
Non-Patent Document 9: Allain F et al., Proc Natl Acad Sci U S A. 2002 Mar 5;99(5):2714-9.
Non-Patent Document 10: Yurchenko V et al., J Biol Chem. 2002 Jun 21;277(25):22959-65.
Non-Patent Document 11: Marcant A et al., J Immunol. 2012 Aug 15;189(4):2023-32.
Non-Patent Document 12: Smith T et al., J Biol Chem. 1995 Aug 4;270(31):18323-8.
Non-Patent Document 13: Barnes AM et al., N Engl J Med. 2010 Feb 11;362(6):521-8.

Summary of Invention

Problems to Be Solved by Invention

One of the objects of the present invention is to provide a novel pharmaceutical composition effective for treating cerebral infarction.

Means for Solving Problems

The present inventors have examined the physiological activity of various in vivo proteins. As a result, they have found that peptidyl-prolyl isomerase B (hereinafter also referred to as PPIB) exhibits therapeutic effects on cerebral infarction in an animal model. Accordingly, the present invention provides, in one aspect, a pharmaceutical composition comprising PPIB protein for the treatment of cerebral infarction.

That is, the present invention includes in some aspects:

(1) a pharmaceutical composition for treating cerebral infarction, comprising a peptidyl-prolyl isomerase B (PPIB) protein, or a cell which secretes the PPIB protein;
(2) the pharmaceutical composition of (1), wherein the PPIB protein is:

a) a protein comprising the amino acid sequence of SEQ ID NO:3;
b) a protein comprising an amino acid sequence in which one or more amino acids are substituted, inserted, deleted and/or added in the amino acid sequence of SEQ ID NO:3;
c) a protein comprising an amino acid sequence having 80% or more sequence identity with the amino acid sequence of SEQ ID NO:3;
d) a protein encoded by the DNA which hybridizes under stringent conditions with the nucleic acid sequence of SEQ ID NO:4;
e) a protein encoded by a nucleic acid sequence having 70% or more sequence identity with the nucleic acid sequence of SEQ ID NO:4;
f) a protein comprising an amino acid sequence in which a signal sequence is removed from an amino acid sequence of a homologue of a protein comprising the amino acid sequence of SEQ ID NO:1; or
g) a protein comprising an amino acid sequence in which a signal sequence is removed from an amino acid sequence of an orthologue of a protein comprising the amino acid sequence of SEQ ID NO:1, wherein the protein has 80% or more sequence identity with the amino acid sequence of SEQ ID NO:3;

(3) a pharmaceutical composition for treating cerebral infarction, comprising a nucleic acid encoding PPIB protein;
(4) the pharmaceutical composition of (3), wherein the nucleic acid encoding PPIB protein is:

i) a nucleic acid comprising the nucleic acid sequence of SEQ ID NO:4;
ii) a nucleic acid which hybridizes under stringent conditions with the nucleic acid sequence set forth in SEQ ID NO:4; or
iii) a nucleic acid having 70% or more sequence identity with the nucleic acid sequence of SEQ ID NO:4.

Brief Description of Drawings

FIG. 1 is a graph showing weight change of each group of mouse. Data are shown as mean ± standard error (N = 8).
FIG. 2 is a graph showing the therapeutic effect of PPIB protein on cerebral infarction due to transient cerebral ischemia in mouse. The right middle cerebral artery of the mouse was blocked for 60 minutes followed by reperfusion, the brain was removed 2 days after the reperfusion, and the cerebral infarction volume was calculated by TTC staining. Vehicle or test substance was administered intravenously 6 and 24 hours after the reperfusion (total 2 times). The value is shown as mean ± standard error (N = 8). ** Significantly different from vehicle group at $p < 0.01$ (one-way ANOVA).

Mode for Carrying Out Invention

In one aspect, the present invention provides a pharmaceutical composition for treating cerebral infarction comprising PPIB protein, a nucleic acid encoding PPIB protein, or a cell secreting PPIB protein.
Human PPIB is a protein consisting of full-length 216 amino acids (SEQ ID NO:1). In the living body, a signal sequence (amino acid position 1-33 of SEQ ID NO:1) is removed in the process of being extracellularly secreted. And, the mature protein (SEQ ID NO:3) consisting of an amino acid sequence corresponding to the amino acid position 34-216 of SEQ ID NO:1 is considered to exert a function.
Examples of PPIB protein include, but are not limited to, a protein comprising or consisting of the amino acid sequence of SEQ ID NO:3. In one embodiment, PPIB protein also includes a protein functionally equivalent to the protein comprising or consisting of the amino acid sequence of SEQ ID NO:3. Such a protein includes, for example:

a) a protein comprising or consisting of the amino acid sequence of SEQ ID NO:3 in which one or more amino acids, for example, 1 to about 40, 1 to about 30, 1 to about 20, 1 to about 10, 1 to about 5, for example, one, two, three, four or five, or 1 to 4, for example, one, two, three or four amino acids are substituted, inserted, deleted and/or added, wherein the protein is functionally equivalent to the protein comprising or consisting of the amino acid sequence of SEQ ID NO:3;
b) a protein comprising or consisting of an amino acid sequence having about 80% or more, for example about 85% or more, about 90% or more, about 91% or more, about 92% or more, about 93% or more, about 94% or more, about 95% or more, about 96% or more, about 97% or more, about 98% or more, or about 99% or more sequence identity with the amino acid sequence of SEQ ID NO:3, wherein the protein is functionally equivalent to the protein comprising or consisting of the amino acid sequence of SEQ ID NO:3;
c) a protein encoded by the DNA which hybridizes under stringent conditions with the nucleic acid sequence of SEQ ID NO:4, wherein the protein is functionally equivalent to the protein comprising or consisting of the amino acid sequence of SEQ ID NO:3;
d) a protein encoded by a nucleic acid sequence having about 70% or more, for example, 75% or more, about 80% or more, about 90% or more, about 95% or more, about 97% or more, about 98%, or more or about 99% or more sequence identity with the nucleic acid sequence of SEQ ID NO:4, wherein the protein is functionally equivalent to the protein comprising or consisting of the amino acid sequence of SEQ ID NO:3;
e) a protein comprising or consisting of an amino acid sequence in which a signal sequence is removed from an amino acid sequence of a homologue (i.e., orthologue or paralogue) of a protein comprising or consisting of the amino acid sequence of SEQ ID NO:1 (human PPIB), wherein the protein is functionally equivalent to the protein comprising or consisting of the amino acid sequence of SEQ ID NO:3;
f) a protein comprising or consisting of an amino acid sequence in which a signal sequence has been removed from an amino acid sequence of an orthologue of the protein comprising or consisting of the amino acid sequence of SEQ ID NO:1 (human PPIB), wherein the protein has about 80% or more, for example about 85% or more, about 90% or more, about 91% or more, about 92% or more, about 93% or more, about 94% or more, about 95% or more, about 96% or more, about 97% or more, about 98% or more, or about 99% or more sequence identity with the amino acid sequence of SEQ ID NO:3, and wherein the protein is functionally equivalent to the protein comprising or consisting of the amino acid sequence of SEQ ID NO:3.

The protein comprising or consisting of the amino acid sequence of SEQ ID NO:3 has a cysteine residue. Therefore, in one embodiment, a cysteine residue may be substituted with another amino acid for the purpose of, for example, stabilization of properties and conformation during production and purification of the protein, or suppression of intermolecular disulfide bond.

Examples of the nucleic acid encoding PPIB protein include, but are not limited to, a nucleic acid comprising or consisting of the nucleic acid sequence of SEQ ID NO:4. In certain embodiment, the nucleic acid encoding PPIB protein also includes a nucleic acid functionally equivalent to the nucleic acid comprising or consisting of the nucleic acid sequence of SEQ ID NO:4. Such nucleic acids include, for example:

a) a nucleic acid which hybridizes under stringent conditions with the nucleic acid sequence of SEQ ID NO:4;
b) a nucleic acid having about 70% or more, for example about 75% or more, about 80% or more, about 85% or more, about 90% or more, about 95% or more, about 97% or more, about 98% or more, or about 99% or more sequence identity with the nucleic acid sequence of SEQ ID NO:4.

The identity of base sequences and amino acid sequences may be determined using a homology search site via the internet (For example, homology searches such as FASTA, BLAST, PSI-BLAST, and SSEARCH may be used in the European Bioinformatics Institute (EBI): http://www.ebi.ac.uk/Tools/sss/). In addition, a search using BLAST may be carried out through the web site of the National Center for Biotechnology Information (NCBI) (examples of which include BLAST page at the homepage of NCBI website; https://blast.ncbi.nlm.nih.gov/Blast.cgi; Altschul, S.F. et al., J. Mol. Biol., 1990, 215(3): 403-10; Altschul, S.F. & Gish, W., Meth. Enzymol., 1996, 266: 460-480; Altschul, S.F. et al., Nucleic Acids Res., 1997, 25: 3389-3402)).

As used herein, "nucleic acid" is a molecule in which nucleotides are polymerized, and includes oligonucleotide, or polynucleotide. In addition, it includes single-stranded or double-stranded DNA. Further, it also includes those formed only with natural nucleotides, and those containing non-natural base, nucleotide, nucleoside in part, or synthetic nucleic acid. Typically, the nucleic acid is DNA.

As used herein, "stringent conditions" refers to, as the specific example, hybridization with 6×SSC, 40% formamide at 25°C and washing with 1×SSC at 55°C. The stringency depends on conditions such as salt concentration, formamide concentration, or temperature; however it is obvious for those skilled in the art to set these conditions so as to obtain a necessary stringency. For example, see Sambrook et al., "Molecular Cloning: A Laboratory Manual"(1989). For example, DNA encoding a PPIB protein homologue other than the protein comprising or consisting of the amino acid sequence of SEQ ID NO:3 is able to be isolated by using hybridization.

As used herein, "functionally equivalent" to the protein comprising or consisting of the amino acid sequence of SEQ ID NO:3 refers to exhibiting the same medicinal effect as the protein in an animal model of cerebral infarction, where the "same" means to be the same in qualitative evaluation. Examples of a protein functionally equivalent to the protein comprising or consisting of the amino acid sequence of SEQ ID NO:3 include a protein having the same medicinal effect as the protein comprising or consisting of the amino acid sequence of SEQ ID NO:3 (For example, the effect of reducing cerebral infarction), and showing at least about 10%, for example, about 10% or more, about 20% or more, about 30% or more, about 40% or more, about 50% or more, about 60% or more, about 70% or more, about 80% or more, about 90% or more, about 95% or more, or about 100% or more medicinal effect as compared to the protein comprising or consisting of the amino acid sequence of SEQ ID NO:3 in a quantitative evaluation.

PPIB protein, or a protein functionally equivalent thereto may be a protein subjected to various modifications such as physiological modification with sugar chain, labeling with fluorescence or radioactive substance, or fusion with another protein. In addition, for the purpose of stabilization of protein properties and structure during production and purification, suppression of intermolecular disulfide bond etc., oxidation of -SH group in cysteine residue (for example, conversion to sulfo group), glutathionylation, nitrosylation, alkylation, conjugating to maleimide, etc. may be carried out. Any of them may be used as PPIB protein as long as they are functionally equivalent to PPIB.

Examples of a method for obtaining PPIB protein include, but not limited to, recombinant expression (mammalian cell, yeast, E. coli, insect cell, etc.), synthesis using a cell-free system, extraction from a culture supernatant of non-transfected cell, and purchase of commercially available product. Examples include a method of introducing into a host cell a nucleic acid having a nucleic acid sequence encoding a signal sequence according to the host and the nucleic acid sequence of SEQ ID NO:4 and recovering from culture supernatant after recombinant expression, a method of introducing into a host cell a nucleic acid having the nucleic acid sequence of SEQ ID NO:4 and disrupting the cell and recovering after recombinant expression, and a method of synthesizing in a cell-free system using a nucleic acid having the nucleic acid sequence of SEQ ID NO:4 as a template. A cell used for these methods is not particularly limited, and includes, for example, HEK293 cell.

The obtained PPIB protein may be isolated from inside or outside (such as medium) of the host cell, and may be purified as protein which is substantially pure and homogenous. A method for isolating and purifying protein is not particularly limited, and may be performed using an isolation and purification method used in the conventional purification of protein. For example, protein may be isolated and purified by appropriately selecting and/or combining a chromatography column, filter, ultrafiltration, salting out, solvent precipitation, solvent extraction, distillation, immunoprecipitation, SDS-polyacrylamide gel electrophoresis, isoelectric focusing, dialysis, recrystallization, etc.

Examples of chromatography include affinity chromatography, ion-exchange chromatography, hydrophobic chromatography, gel filtration, reverse phase chromatography, and adsorption chromatography (Marshak et al., Strategies for Protein Purification and Characterization: A Laboratory Course Manual. Ed Daniel R. Cold Spring Harbor Laboratory Press, 1996). The chromatography may be performed using liquid phase chromatography such as HPLC and FPLC.

In addition, PPIB protein is preferably substantially purified protein. As used herein, "substantially purified" means that the purity of the PPIB protein (proportion of PPIB protein in total protein components) is about 50% or more, about 60% or more, about 70% or more, about 80% or more, about 90% or more, about 95% or more, 100% or close to 100%. The upper limit for "close to 100%" depends on the purification technique and analytical technique of those skilled in the art, and the examples include 99.999%, 99.99%, 99.9%, and 99%.

Moreover, a substantially purified protein includes any protein purified by any purification method as long as the protein purity is as mentioned above. Examples include, but are not limited to, protein substantially purified by appropriately selecting and/or combining the above-mentioned chromatography column, filter, ultrafiltration, salting out, solvent precipitation, solvent extraction, distillation, immunoprecipitation, SDS-polyacrylamide gel electrophoresis, isoelectric focusing, dialysis, recrystallization, etc.

A form of PPIB protein, a nucleic acid encoding PPIB protein, or a cell secreting PPIB protein as an active ingredient of the pharmaceutical composition of the present aspect is not particularly limited, and examples thereof include PPIB protein, a vector into which DNA encoding PPIB protein is inserted, and a cell introduced with a vector into which DNA encoding PPIB protein is inserted.

A cell secreting PPIB protein may be produced, for example, by producing a vector via insertion of a DNA in which a DNA encoding secretion signal is linked to a DNA encoding PPIB protein into a known expression vector or a gene therapy vector and introducing the vector into mammalian cells such as fibroblasts (such as normal skin fibroblast and cell line derived therefrom), insect cells, and other cells. Examples of the DNA encoding secretion signal include, but not limited to, a DNA having the nucleic acid sequence of SEQ ID NO:5. Although there is no particular limitation in animal species from which these cells are derived, it is preferable to use a cell from animal species of the subject of vector administration, a cell from the subject itself, or a cell derived from a blood relative of the subject of vector administration.

DNA encoding PPIB protein may be cDNA, genomic DNA, natural DNA, or artificially-synthesized DNA so long as the DNA encodes PPIB protein. DNA encoding PPIB protein is contained in a pharmaceutical composition of the present aspect, typically, in the form of vector (such as gene therapy vector) into which the DNA is inserted.

Examples of the gene therapy vector include, but are not limited to, plasmid vector, retrovirus vector, lentivirus vector, adenovirus vector, adeno-associated virus vector, Sendai virus vector, Sendai virus envelope vector, and papilloma virus vector. The gene therapy vector may contain promoter DNA sequence which effectively induces gene expression, a factor which regulates gene expression, and a molecule which is necessary for maintaining DNA stability.

A pharmaceutical composition of the present aspect may be formulated according to the usual method (for example, see Remington's Pharmaceutical Science, latest edition, Mark Publishing Company, Easton, U.S.A), and may contain pharmaceutically acceptable carrier and additive together. Examples thereof include surfactant, excipient, colorant, perfume, preservative, stabilizer, buffer, suspending agent, isotonizing agent, binder, disintegrant, lubricant, flow promoter, and flavoring agent, although they are not limited thereto and other common carriers may be appropriately used. Specific examples include light anhydrous silicic acid, lactose, crystalline cellulose, mannitol, starch, carmellose calcium, carmellose sodium, hydroxypropylcellulose, hydroxypropylmethylcellulose, polyvinylacetaldiethylamino acetate, polyvinylpyrrolidone, gelatin, medium-chain fatty acid triglyceride, polyoxyethylene hydrogenated castor oil 60, white sugar, carboxymethyl cellulose, corn starch, and inorganic salts.

A method for administering the pharmaceutical composition of the present aspect includes oral or parenteral administration. Specific examples of the administration method include administration by injection, transnasal administration, transpulmonary administration, and percutaneous administration. Examples of administration by injection include intravenous injection, intramuscular injection, intraperitoneal injection, and hypodermic injection, by which the pharmaceutical composition of the present aspect may be administered systemically or locally (such as subcutaneously or intracutaneously, or to the skin surface, eyeball, palpebral conjunctiva, nasal mucosa, intraoral mucosa, gastrointestinal mucosa, vaginal/intrauterine mucosa, or lesion site).

The method of administration may be appropriately selected based on, such as age and symptoms of the patient. When PPIB protein is administered, the dose per time of the protein may be selected within a range of about 0.0000001 mg to about 1000 mg per kg body weight of a patient. Alternatively, the dose may be selected, for example, within a range of about 0.00001 mg to about 100000 mg per body of patient. When administering a cell secreting PPIB protein or gene therapy vector inserted with DNA encoding PPIB protein, they may be administered such that the amount of PPIB protein in the damaged tissue is within the above range. However, the dosage of the pharmaceutical composition of the present aspect is not limited thereto.

As used herein, "patient" or "subject" includes human or non-human animal, and examples of the animal may include, but not limited to, human, mouse, rat, monkey, pig, dog, dog, rabbit, hamster, and guinea pig.

As used herein, "treatment" refers to, for example, any of the palliation, improvement, and/or elimination, alleviation and/or stabilization (e.g., stop to progress to a more advanced stage) of the symptom.

The present invention further provides the following aspects:

(1) a method for treating cerebral infarction, comprising administering to a subject PPIB protein, a nucleic acid encoding PPIB protein, or a cell secreting PPIB protein;
(2) PPIB protein, a nucleic acid encoding PPIB protein, or a cell secreting PPIB protein, for use in the treatment of cerebral infarction;
(3) use of PPIB protein, a nucleic acid encoding PPIB protein, or a cell secreting PPIB protein in the manufacture of a medicament for the treatment of cerebral infarction.

In these aspects, PPIB protein, a functionally equivalent protein thereof, the nucleic acid encoding PPIB protein, the cell secreting PPIB protein, and form, formulation, administration method, and dosage thereof and other embodiments thereof are as described above.

The prior art documents cited herein are hereby incorporated by reference in their entirety. In the event of inconsistency between the description of the present specification and the cited documents, it is adjusted by the description of the present specification.

Hereinafter, the present invention will be more specifically described by way of examples, but these examples are not necessarily intended to limit the present invention.

## EXAMPLES

### Example 1: Effect of human PPIB on mouse cerebral infarction

Materials

Animal:

C57BL/6N male mice (microbial grade: SPF) were purchased from CLEA Japan, Inc. (Tokyo, Japan) at 7 weeks of age and used at 23 to 25 g after being acclimated in the animal breeding room for 3 days or more. The animals were raised in an animal breeding room set at room temperature 22 ± 2°C, humidity 50% ± 20%, and illumination time 12 hours (7 am to 7 pm). They were automatically fed with solid feed, CE-2 (Oriental Yeast Co., Ltd.) and tap water, and raised at 3 animals per cage.

Drug:

Human peptidyl-prolyl isomerase B (PPIB) was used as a test substance. The human PPIB was obtained by introducing a vector inserted with a DNA having the nucleic acid sequence of SEQ ID NO:2 into a HEK293 cell, and purifying the supernatant from the culture of the cell. The human PPIB thus obtained is a protein consisting of the amino acid sequence of SEQ ID NO:3 because it is a mature protein produced by the removal of signal sequence from a protein consisting of the amino acid sequence of SEQ ID NO:1 synthesized in the cell during its extracellular secretion. It was confirmed that the human PPIB was a protein consisting of the amino acid sequence of SEQ ID NO:3 by peptide sequencing at the N-terminal and mass spectrometry (MALDI-TOF/TOF). Normal saline (Otsuka Normal Saline, Otsuka Pharmaceutical Co., Ltd.) was used as a vehicle. The test substance was dissolved in Otsuka Distilled Water (Otsuka Pharmaceutical Co., Ltd.) to a concentration of 1 mg/ml, aliquoted in 50 μl portions, and stored at -30°C until use. Before administering the test substance to mice, the stored test substance was dissolved, and 200 μL of Otsuka Normal Saline was added and diluted to the desired concentration (0.2 mg/mL). The dosing solution was stored on ice until dosing.

Equipment:

An embolic thread was prepared by rounding the tip of 20 mm long No. 6 nylon thread (Nescosuture 6-0 nylon) with a soldering iron, and coating for 7 mm from the tip to a diameter 0.21 to 0.23 mm with a silicone resin (Dow Corning, RTV sealant 732).

Test method

Transient cerebral ischemia:

C57BL/6N male mouse was anesthetized with isoflurane (Abbott Japan) using an anesthesia machine (MK-A110D, Muromachi Kikai Co., Ltd.). Under anesthesia (3% at the time of introduction, 2% maintenance), the right carotid artery was dissected by median incision, and the common carotid artery and the external carotid artery were stringed, and then the blood flow of the common carotid artery and the external carotid artery was temporarily blocked. The embolic thread was inserted from the external carotid artery, and the external carotid artery was cut. Then the embolic thread was inserted into the internal carotid artery for 10.5 mm from the bifurcation of the external carotid artery and the internal carotid artery to block the origin of the middle cerebral artery. And then, the surgical wound was closed. The rectum temperature of the mouse was maintained at 36.5-37.5°C using a heat mat during surgery at the time of preparing cerebral infarction. The ischemic mouse was returned to the cages and allowed to be freely raised. The embolic thread was withdrawn 60 minutes after the infarct under anesthesia and the incision was closed. Rectal temperature was not adjusted during the reperfusion. The surgery was performed under a microscope (POM-50 II, Konan Medical).

Weight measurement:

Body weight were measured before, and 1 and 2 days after surgery.

Administration:

The test substance or the vehicle was administered twice in total after 6 and 24 hours of ischemia reperfusion. The dosing solution was filled into a 1 mL syringe, and administered at 0.2 mL volume from the tail vein using a 27G injection needle.

Measurement of cerebral infarction area:

Two days after ischemia reperfusion, the mouse was exsanguinated and killed by abdominal aortic section under anesthesia, and the brain was collected. The collected brain was subjected to a treatment with a mouse brain slicer (Zivic Instruments) to prepare four cross-sections of 2 mm in thickness from the border of the cerebrum and cerebellum. They were stained with 1% 2,3,5-triphenyltetrazolium chloride (TTC) (Sigma) for 30 minutes at 37°C. The brain section was imaged using a microscope (magnification x 1.5, Olympus), and infarct size was determined using image analysis software (Photoshop CS5, Adobe), and for each brain section, left brain area, right brain non-impaired area and right brain injury area were determined. The whole brain area was calculated as follows: whole brain area = left brain area × 2.

The volume of cerebral infarction was calculated by the following equation based on the method of determining the volume of the frustum:

$$[s + S + \sqrt{(sS)}] \times h / 3$$

(where s: area of segment 1, S: area of segment 2, h: distance between segments).

Three volumes between adjacent sections were determined and summed to calculate the percentage of cerebral infarction volume:

Cerebral infarction volume (%) = cerebral infarction volume / whole brain volume x 100.

Statistical processing:

The measured value was expressed as mean value and standard error (mean ± SEM). The measured value was statistically analyzed by one-way analysis of variance using spreadsheet software, and $p < 0.05$ was considered to indicate a significant difference.

Test results

The mice were divided into groups after cerebral ischemia reperfusion, and none of the mice died in the period until the brain was removed 2 days after administration of the vehicle or PPIB, respectively. The change in mouse weight in each group during this period is shown in FIG. 1. The weights of mice used for surgery did not differ between the groups. After surgery, the weight of the mice decreased, and both groups decreased by about 3 g on one day after surgery and about 5 g on two days after surgery.

The brain was removed on two days after transient cerebral ischemia, and the volume of the TTC-stained cerebral infarct was measured, and the results are shown in FIG. 2. The infarct volume relative to the whole brain volume was 20.6% in the vehicle group and 10.9% in the PPIB group, showing a significant decrease of 47.2%. From the above results, it was shown that PPIB exerts a therapeutic effect on cerebral infarction due to transient cerebral ischemia.

Industrial Applicability

The thrombolytic agent t-PA is an existing medicine which is effective in the treatment of cerebral infarction. However, t-PA is limited its use, for example, it is non-approved in the cases that: more than 4.5 hours post-cerebral infarction; a patient has a history of nontraumatic intracranial hemorrhage; a thoracic aortic dissection is strongly suspected; and extensive early ischemic changes are observed on CT and MRI. In addition, it is estimated that 5 to 6% of all patients with cerebral infarction are actually applied t-PA in Japanese medical practice. Thus, there is no effective treatment available for most patients. PPIB is considered to exert therapeutic effects on cerebral infarction by a mechanism different from t-PA. Thus, the therapeutic agent for cerebral infarction comprising PPIB provided by the present application provides effective treatment for patients for whom t-PA is not applicable. Further, it is also expected to provide great benefits to patients for whom t-PA alone does not give a sufficient therapeutic effect, by administration of PPIB alone or in combination with t-PA.

## Claims

**1.** A pharmaceutical composition for treating cerebral infarction, comprising a peptidyl-prolyl isomerase B (PPIB) protein, or a cell which secretes PPIB protein.

**2.** The pharmaceutical composition according to claim 1, wherein the PPIB protein is:

a) a protein comprising the amino acid sequence of SEQ ID NO:3;
b) a protein comprising an amino acid sequence in which one or more amino acids are substituted, inserted, deleted and/or added in the amino acid sequence of SEQ ID NO:3;
c) a protein comprising an amino acid sequence having 80% or more sequence identity with the amino acid sequence of SEQ ID NO:3;
d) a protein encoded by the DNA which hybridizes under stringent conditions with the nucleic acid sequence of SEQ ID NO:4;
e) a protein encoded by a nucleic acid sequence having 70% or more sequence identity with the nucleic acid sequence of SEQ ID NO:4;
f) a protein comprising an amino acid sequence in which a signal sequence has been removed from an amino acid sequence of a homologue of a protein comprising the amino acid sequence of SEQ ID NO:1; or
g) a protein comprising an amino acid sequence in which a signal sequence has been removed from an amino acid sequence of an orthologue of a protein comprising the amino acid sequence of SEQ ID NO:1, wherein the protein has 80% or more sequence identity with the amino acid sequence of SEQ ID NO:3.

**3.** A pharmaceutical composition for treating cerebral infarction, comprising a nucleic acid encoding PPIB protein.

**4.** The pharmaceutical composition according to claim 3, wherein the nucleic acid encoding PPIB protein is:

i) a nucleic acid comprising the nucleic acid sequence of SEQ ID NO:4;
ii) a nucleic acid which hybridizes under stringent conditions with the nucleic acid sequence of SEQ ID NO: 4; or
iii) a nucleic acid having 70% or more sequence identity with the nucleic acid sequence of SEQ ID NO:4.

[Fig. 1]

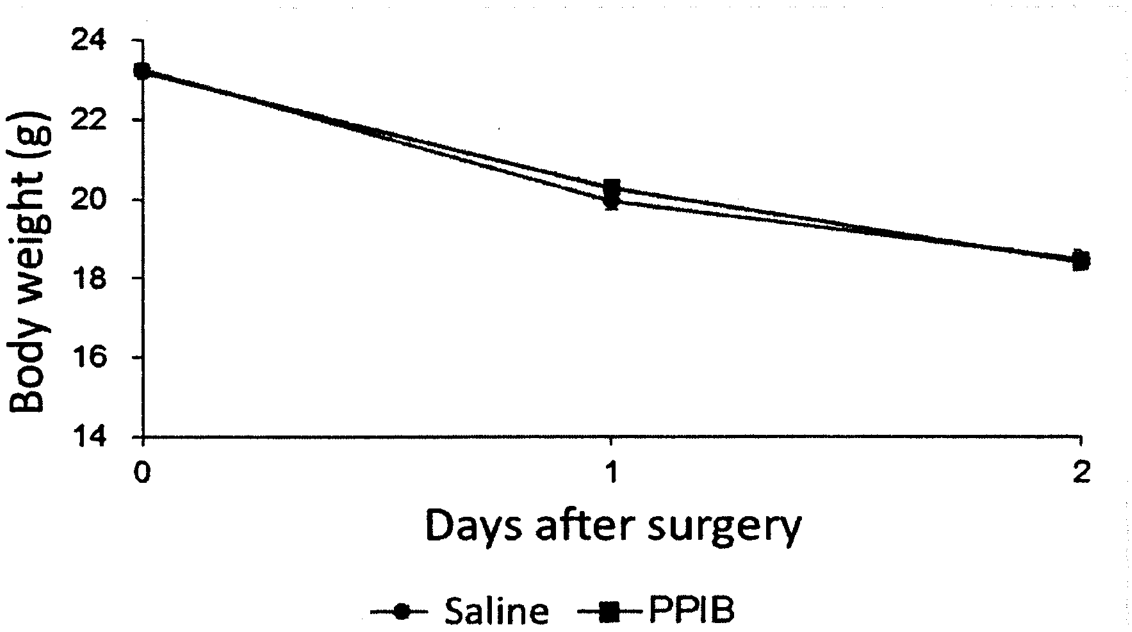
24
22
20
18
16
14
Body weight (g)
0
1
2
Days after surgery
Saline
PPIB

[Fig. 2]

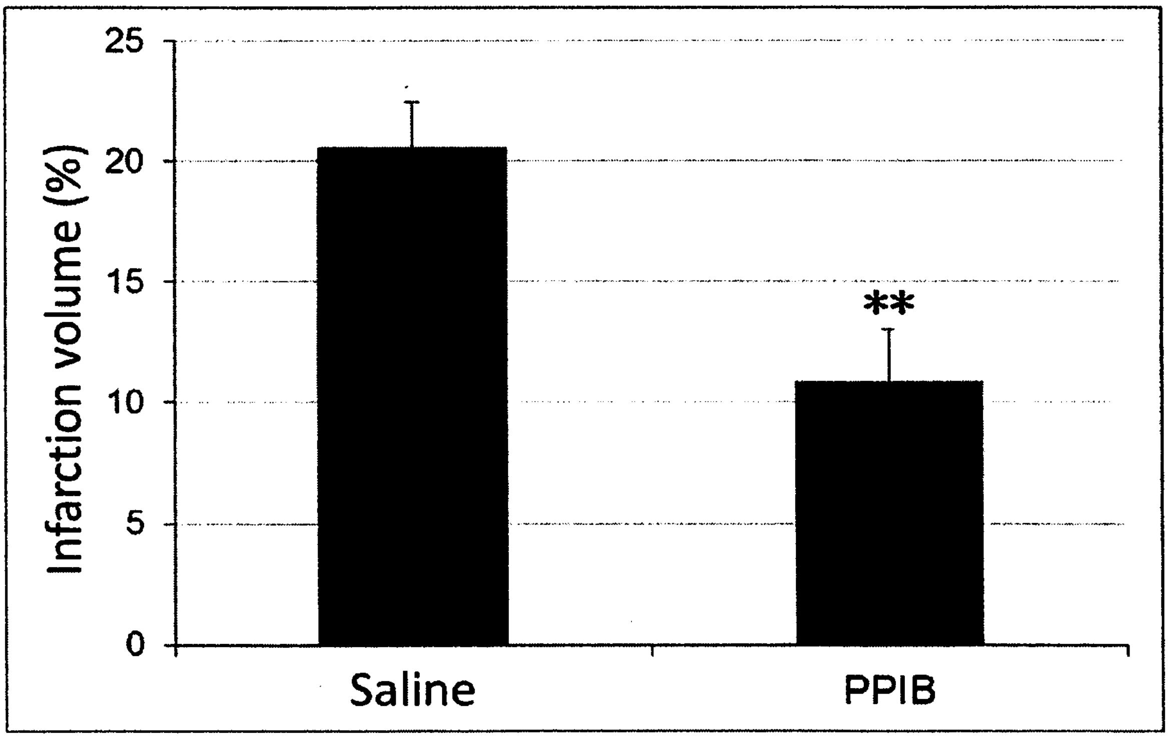
25
20
15
10
5
0
Infarction volume (%)
**
Saline
PPIB

## INTERNATIONAL SEARCH REPORT

International application No.
PCT/JP2017/040395

A. CLASSIFICATION OF SUBJECT MATTER
Int.Cl. A61K38/52(2006.01)i, A61K35/12(2015.01)i, A61K35/76(2015.01)i, A61K35/761(2015.01)i, A61K48/00(2006.01)i, A61P9/10(2006.01)i

According to International Patent Classification (IPC) or to both national classification and IPC

B. FIELDS SEARCHED

Minimum documentation searched (classification system followed by classification symbols)
Int.Cl. A61K38/00-38/58, A61K41/00-45/08, A61K48/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2017 |
| Registered utility model specifications of Japan | 1996-2017 |
| Published registered utility model applications of Japan | 1994-2017 |

Electronic data base consulted during the international search (name of data base and, where practicable, search terms used)
JSTPlus/JMEDPlus/JST7580 (JDreamIII)
CAplus/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| Y | JP 2008-530029 A (THE UNIVERSITY OF WESTERN AUSTRALIA) 07 August 2008, claims 10, 15, 28-31, paragraphs [0017]-[0019], [0092]-[0104], [0144], [0147], [0214], [0235], [0239] & US 2008/0145340 A1, claims 10, 15, 28-31, page 5, line 22 to page 6, line 9, page 26, line 1 to page 29, line 16, page 40, line 20 to page 41, line 2, page 41, line 25 to page 42, line 8, page 61, lines 1-12, page 67, lines 17-27, page 68, lines 21-26, & WO 2006/084333 A1 | 1-4 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04 December 2017 (04.12.2017) | 12 December 2017 (12.12.2017) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2017/040395

C (Continuation). DOCUMENTS CONSIDERED TO BE RELEVANT

| Category* | Citation of document, with indication, where appropriate, of the relevant passages | Relevant to claim No. |
|---|---|---|
| Y | MARK, H. B., et al., 虚血性脳卒中, メルクマニュアル, vol. 18, 2007, pp. 1903-1908, ISBN: 978-4-8222-0398-6, in particular, page 1903, right column, lines 8-9, page 1904, left column, lines 7-8, (Merck Manual), non-official translation ("ischemic stroke") | 1-4 |
| Y | YURCHENKO, et al., "CD147 Is a Signaling Receptor for Cyclophilin B, Biochemical and Biophysical Research Communications", 2001, vol. 288, pp. 786-788, ISSN: 0006-291X, in particular, abstract, page 787, left column, lines 28-39, page 788, right column, lines 5-8, fig. 1 | 1-4 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Summary of Population Survey Report (Definite). Ministry of Health, Labour and Welfare, 2015
- *Neurological Surgery,* December 2015, vol. 43 (12), 1055-1070
- **PRICE ER et al.** *Proc Natl Acad Sci U S A.,* 01 March 1991, vol. 88 (5), 1903-7
- **SPIK G et al.** *J Biol Chem.,* 15 June 1991, vol. 266 (17), 10735-8
- **HASEL KW et al.** *Mol Cell Biol.,* July 1991, vol. 11 (7), 3484-91
- **LUBAN J et al.** *Cell,* 18 June 1993, vol. 73 (6), 1067-78
- **LIN SY et al.** *Exp Cell Res.,* 15 October 2008, vol. 314 (17), 3107-17
- **YURCHENKO V et al.** *Biochem Biophys Res Commun.,* 09 November 2001, vol. 288 (4), 786-8
- **ALLAIN F et al.** *Proc Natl Acad Sci U S A.,* 05 March 2002, vol. 99 (5), 2714-9
- **YURCHENKO V et al.** *J Biol Chem.,* 21 June 2002, vol. 277 (25), 22959-65
- **MARCANT A et al.** *J Immunol.,* 15 August 2012, vol. 189 (4), 2023-32
- **SMITH T et al.** *J Biol Chem.,* 04 August 1995, vol. 270 (31), 18323-8
- **BARNES AM et al.** *N Engl J Med.,* 11 February 2010, vol. 362 (6), 521-8
- **ALTSCHUL, S.F. et al.** *J. Mol. Biol.,* 1990, vol. 215 (3), 403-10, https://blast.ncbi.nlm.nih.gov/Blast.cgi
- **ALTSCHUL, S.F. ; GISH, W.** *Meth. Enzymol.,* 1996, vol. 266, 460-480
- **ALTSCHUL, S.F. et al.** *Nucleic Acids Res.,* 1997, vol. 25, 3389-3402
- **SAMBROOK et al.** Molecular Cloning: A Laboratory Manual. 1989
- **MARSHAK et al.** Strategies for Protein Purification and Characterization: A Laboratory Course Manual. Cold Spring Harbor Laboratory Press, 1996
- Remington's Pharmaceutical Science. Mark Publishing Company

SEQUENCE LISTING

<110> GENOMIX CO., LTD.
OSAKA UNIVERSITY

<120> THERAPEUTIC AGENT FOR CEREBRAL INFARCTION

<130> 673792

<150> JP 2016-220805
<151> 2016-11-11

<160> 5

<170> PatentIn version 3.5

<210> 1
<211> 216
<212> PRT
<213> Homo sapiens

<400> 1

```
Met Leu Arg Leu Ser Glu Arg Asn Met Lys Val Leu Leu Ala Ala Ala
1               5                   10                  15

Leu Ile Ala Gly Ser Val Phe Phe Leu Leu Leu Pro Gly Pro Ser Ala
            20                  25                  30

Ala Asp Glu Lys Lys Lys Gly Pro Lys Val Thr Val Lys Val Tyr Phe
        35                  40                  45

Asp Leu Arg Ile Gly Asp Glu Asp Val Gly Arg Val Ile Phe Gly Leu
    50                  55                  60

Phe Gly Lys Thr Val Pro Lys Thr Val Asp Asn Phe Val Ala Leu Ala
65                  70                  75                  80

Thr Gly Glu Lys Gly Phe Gly Tyr Lys Asn Ser Lys Phe His Arg Val
                85                  90                  95

Ile Lys Asp Phe Met Ile Gln Gly Gly Asp Phe Thr Arg Gly Asp Gly
            100                 105                 110

Thr Gly Gly Lys Ser Ile Tyr Gly Glu Arg Phe Pro Asp Glu Asn Phe
        115                 120                 125

Lys Leu Lys His Tyr Gly Pro Gly Trp Val Ser Met Ala Asn Ala Gly
    130                 135                 140

Lys Asp Thr Asn Gly Ser Gln Phe Phe Ile Thr Thr Val Lys Thr Ala
145                 150                 155                 160
```

```
Trp Leu Asp Gly Lys His Val Val Phe Gly Lys Val Leu Glu Gly Met
                165                 170                 175

Glu Val Val Arg Lys Val Glu Ser Thr Lys Thr Asp Ser Arg Asp Lys
            180                 185                 190

Pro Leu Lys Asp Val Ile Ile Ala Asp Cys Gly Lys Ile Glu Val Glu
        195                 200                 205

Lys Pro Phe Ala Ile Ala Lys Glu
    210                 215
```

<210> 2
<211> 651
<212> DNA
<213> Homo sapiens

<400> 2

```
atgctgcgcc tctccgaacg caacatgaag gtgctccttg ccgccgccct catcgcgggg       60

tccgtcttct tcctgctgct gccgggacct tctgcggccg atgagaagaa gaaggggccc      120

aaagtcaccg tcaaggtgta ttttgaccta cgaattggag atgaagatgt aggccgggtg      180

atctttggtc tcttcggaaa gactgttcca aaaacagtgg ataattttgt ggccttagct      240

acaggagaga aaggatttgg ctacaaaaac agcaaattcc atcgtgtaat caaggacttc      300

atgatccagg gcggagactt caccagggga gatggcacag gaggaaagag catctacggt      360

gagcgcttcc ccgatgagaa cttcaaactg aagcactacg ggcctggctg ggtgagcatg      420

gccaacgcag gcaaagacac caacggctcc cagttcttca tcacgacagt caagacagcc      480

tggctagatg gcaagcatgt ggtgtttggc aaagttctag agggcatgga ggtggtgcgg      540

aaggtggaga gcaccaagac agacagccgg gataaacccc tgaaggatgt gatcatcgca      600

gactgcggca agatcgaggt ggagaagccc tttgccatcg ccaaggagta g               651
```

<210> 3
<211> 183
<212> PRT
<213> Homo sapiens

<400> 3

```
Asp Glu Lys Lys Lys Gly Pro Lys Val Thr Val Lys Val Tyr Phe Asp
1               5                   10                  15

Leu Arg Ile Gly Asp Glu Asp Val Gly Arg Val Ile Phe Gly Leu Phe
            20                  25                  30

Gly Lys Thr Val Pro Lys Thr Val Asp Asn Phe Val Ala Leu Ala Thr
        35                  40                  45
```

```
Gly Glu Lys Gly Phe Gly Tyr Lys Asn Ser Lys Phe His Arg Val Ile
    50                  55                  60

Lys Asp Phe Met Ile Gln Gly Gly Asp Phe Thr Arg Gly Asp Gly Thr
65                  70                  75                  80

Gly Gly Lys Ser Ile Tyr Gly Glu Arg Phe Pro Asp Glu Asn Phe Lys
                85                  90                  95

Leu Lys His Tyr Gly Pro Gly Trp Val Ser Met Ala Asn Ala Gly Lys
            100                 105                 110

Asp Thr Asn Gly Ser Gln Phe Phe Ile Thr Thr Val Lys Thr Ala Trp
        115                 120                 125

Leu Asp Gly Lys His Val Val Phe Gly Lys Val Leu Glu Gly Met Glu
    130                 135                 140

Val Val Arg Lys Val Glu Ser Thr Lys Thr Asp Ser Arg Asp Lys Pro
145                 150                 155                 160

Leu Lys Asp Val Ile Ile Ala Asp Cys Gly Lys Ile Glu Val Glu Lys
                165                 170                 175

Pro Phe Ala Ile Ala Lys Glu
            180


<210>  4
<211>  552
<212>  DNA
<213>  Homo sapiens

<400>  4
gatgagaaga agaaggggcc caaagtcacc gtcaaggtgt attttgacct acgaattgga       60

gatgaagatg taggccgggt gatctttggt ctcttcggaa agactgttcc aaaaacagtg      120

gataattttg tggccttagc tacaggagag aaaggatttg gctacaaaaa cagcaaattc      180

catcgtgtaa tcaaggactt catgatccag ggcggagact tcaccagggg agatggcaca      240

ggaggaaaga gcatctacgg tgagcgcttc cccgatgaga acttcaaact gaagcactac      300

gggcctggct gggtgagcat ggccaacgca ggcaaagaca ccaacggctc ccagttcttc      360

atcacgacag tcaagacagc ctggctagat ggcaagcatg tggtgtttgg caaagttcta      420

gagggcatgg aggtggtgcg gaaggtggag agcaccaaga cagacagccg ggataaaccc      480

ctgaaggatg tgatcatcgc agactgcggc aagatcgagg tggagaagcc ctttgccatc      540

gccaaggagt ag                                                          552
```

<210> 5
<211> 99
<212> DNA
<213> Artificial

<220>
<223> An artificially synthesized nucleotide sequence

<400> 5
atgctgcgcc tctccgaacg caacatgaag gtgctccttg ccgccgccct catcgcgggg 60

tccgtcttct tcctgctgct gccgggacct tctgcggcc 99